# EUROPEAN PATENT SPECIFICATION

(11) **EP 0 635 252 B1**
(45) Date of publication and mention of the grant of the patent: **16.01.2002**
(21) Application number: 94202124.7
(22) Date of filing: 20.07.1994
(51) Int. Cl.: A61H 33/00

(54) **Process and plant for washing and/or disinfecting a hydromassage system**
Verfahren und Anlage zur Reinigung und/oder Desinfektion eines Hydromassagesystems
Procédé et installation pour nettoyer et/ou désinfecter un système d'hydromassage

(30) Priority: 22.07.1993 IT MI931624
(43) Date of publication of application: 25.01.1995
(73) Proprietor: Jacuzzi Europe Spa, 33098 Valvasone (Pordenone) (IT)
(72) Inventor: Ianni, Carmelo, I-33034 Fagagna (Udine) (IT); Furlan, Livio, I-33170 Pordenone (IT)
(74) Representative: Agostini, Agostino

(56) References cited:
- EP-A- 0 164 068
- EP-A- 0 215 514
- EP-A- 0 320 696
- EP-A- 0 356 529
- EP-A- 0 455 088
- EP-A- 0 519 572

## Description

The present invention relates to a hydromassage system and more precisely to an improved process and associated improved plant for washing and disinfecting the system.

It is known that hydromassage consists in striking the user's body immersed inside a bath with a jet of pressurised water mixed with air, delivered by one or more nozzles mounted in the walls of the bath.

Various types of delivery nozzles for hydromassage are known, operation of which consists generally in passing a certain quantity of water through a section having the shape of a Venturi tube, causing in this way a negative pressure which in turn causes the suction of air which is mixed with water expelled from the nozzle in the form of a jet.

Various technical requirements are associated with a hydromassage system, including in particular that of washing and/or disinfecting the system itself before (or after, depending on the circumstances) it has been used.

In particular this requirement is of greater importance for hydromassage systems installed in communal premises (hotels, rest homes, gyms, etc.) where the users, doubting the effectiveness of the cleaning operations performed by the service personnel, prefer themselves to activate, as and when required, an automatic device for washing and disinfecting the hydromassage system.

Various washing and disinfecting devices exist in the sector: a first example involves the pumping of disinfectant into the water pipes with the delivery nozzles closed.

Although in general this device performs its function, it is not without drawbacks: in fact the delivery nozzles are not exposed to the action of the circulating disinfectant, the interior of the bath is not involved in any way and the user is unable to check what is happening during the cycle.

A second example involves the introduction of disinfectant into the bath full of water and then the circulation of the treated water inside the hydraulic piping such that it flows back into the bath through the delivery nozzles.

This device is also not without drawbacks: in fact, a large quantity of water is required, since the use of the hydromassage water to carry out disinfecting is not acceptable.

Furthermore, a large quantity of additives is used with consequences both in terms of the costs and from an ecological point of view.

Also, during emptying, any residue present in the water is deposited along the walls of the bath.

In this connection EP-A-0 164 068 can be cited as a prior construction where the suction of additive-containing water by a pump - which recycles it back into the hydromassage bath - for cleaning and/or disinfecting purposes takes place through a suction port which is above the bottom of the bath. Therefore, a certain amount of water is substantially not affected by the recycling and, in any case, a complete cleaning of the bath including the bottom surface thereof, cannot be ensured. Also worth mentioning is EP-A-0 519 572 where a cleaning and/or disinfecting operation is only possible after the hydromassage bath has been filled. This does not guarantee the users about the hygiene of the hydraulic system before taking a hydromassage and furthermore wastes water as well as additives.

The aim of the present invention is to propose a new process and associated plant for washing and/or disinfecting a hydromassage system which does not have the abovementioned drawbacks.

A first aim of the present application, therefore, is a process for washing and/or disinfecting a hydrocassage system according to the appended claim 1.

The present Application also relates to a hydromassage system according to the appended claim 7.

Further aspects of the present invention, as well as positive features and advantages thereof will emerge from the following detailed, but not limiting description, with reference to the accompanying drawings in which:
Figure 1 is a front, partially sectioned view of an embodiment of the hydromassage plant according to the present invention;
Figure 2 is a front, partially sectioned view of a modified embodiment of the plant according to Figure 1.

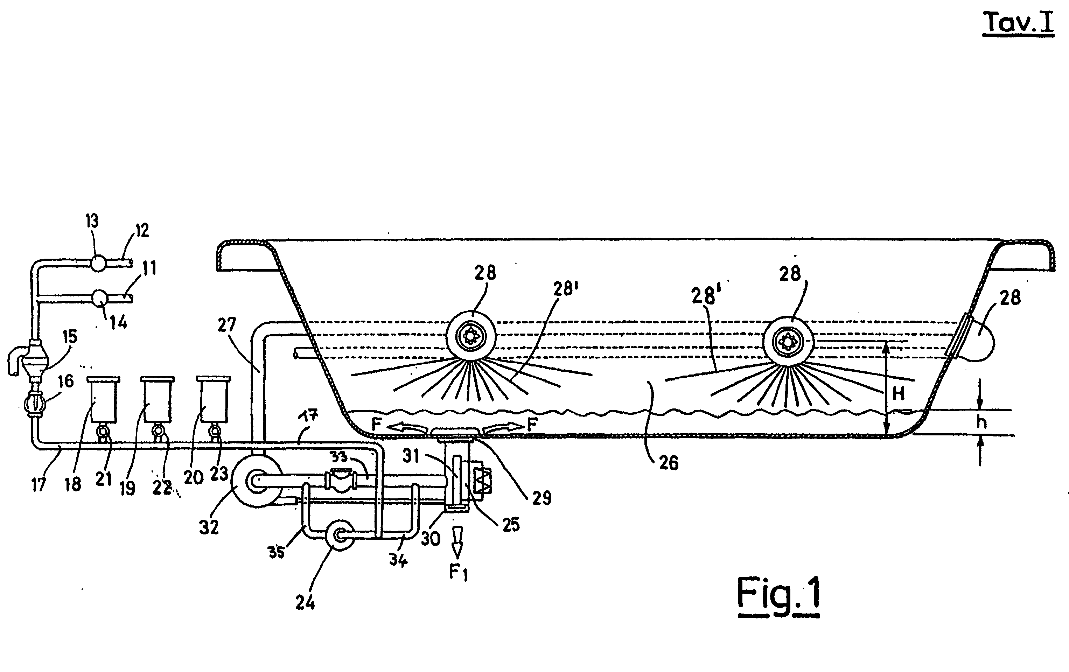

With reference to Figure 1, the plant has two inlets 11 and 12, for cold and hot water respectively, connected to the domestic water mains; said inlets are controlled respectively by the solenoid valves or actuators 13 and 14 which in turn are connected to a timer (not shown) which governs opening and closing of them at predetermined times (sequence and duration).

15 denotes an air-break device of the known type for preventing the penetration of unwanted liquids into the urban water system, said device being protected by a non-return valve 16.

The water supply pipe 17 has connected to it three metering reservoirs 18, 19 and 20 containing respectively a cleaning substance, a disinfecting substance and a scenting substance (said substances are widely available on the market).

Opening/closing of the metering reservoirs is controlled by the solenoid valves 21, 22 and 23 which in turn are connected to a timer (not shown) which also governs predetermined metering of the substances.

The supply pipe 17 is connected to the auxiliary or service pump 24 which can be activated by a timer (not shown) which is connected in turn on one side to the nozzle 25 situated on the bottom of the bath 26 and on the other side to the hydromassage delivery pipe 27, the latter leading to the delivery nozzles 28 arranged on the walls of the bath at a predetermined height H from the bottom of the latter (roughly about halfway up the height of the bath).

The nozzle indicated by 25 is divided, operationally speaking, into two parts: the first part which is defined as an inlet/suction nozzle 29 (normally open during the washing/disinfecting operations), and the second part which is defined as a drainage nozzle 3.0 and is positioned lower down; closing/opening of the drainage nozzle is controlled by the solenoid valve or actuator 31. which in. turn can be activated by means of a timer (not shown).

It should be noted that, although the nozzle 25 has been divided, operationally speaking, into two nozzles 29 and 30 (this will be understood more clearly below from the description of operation of the plant), structurally speaking it still constitutes a single nozzle.

The delivery nozzles 28 may be of any suitable type having generally an internal cavity profiled so as to exploit the principle of the Venturi tube; however, for the construction of the present plant, particular preference is given to the delivery nozzles which are the subject of Patent Applications No. 20112 A/90 of 23 April 1990 and Patent No. 219491 in the name of the same Applicant, the contents of which are to be regarded as an integral part of the present description.

Briefly described, these nozzles are provided with a plurality of service channels for expelling the washing and/or disinfecting liquid, arranged in a fan-like configuration directed preferably towards the bottom of the bath (see direction of the jets 28' in Fig. 1). and directed against the adjacent wall of the bath, being activated by means of a solenoid possibly connected to a timer (not shown).

32 denotes the main pump of the hydromassage system which is connected on one hand to the nozzle 25 directly through an inlet pipe 33 and indirectly through the auxiliary pump 24 and its connection pipes 34,35 and on the other hand to the delivery pipe 27.

This pump, which is normally used for hydromassage, could perform the function of the auxiliary pump 24, by suitably reducing the number of revolutions; in this case, like the auxiliary pump, the main pump can be suitably connected to a timer (not shown).

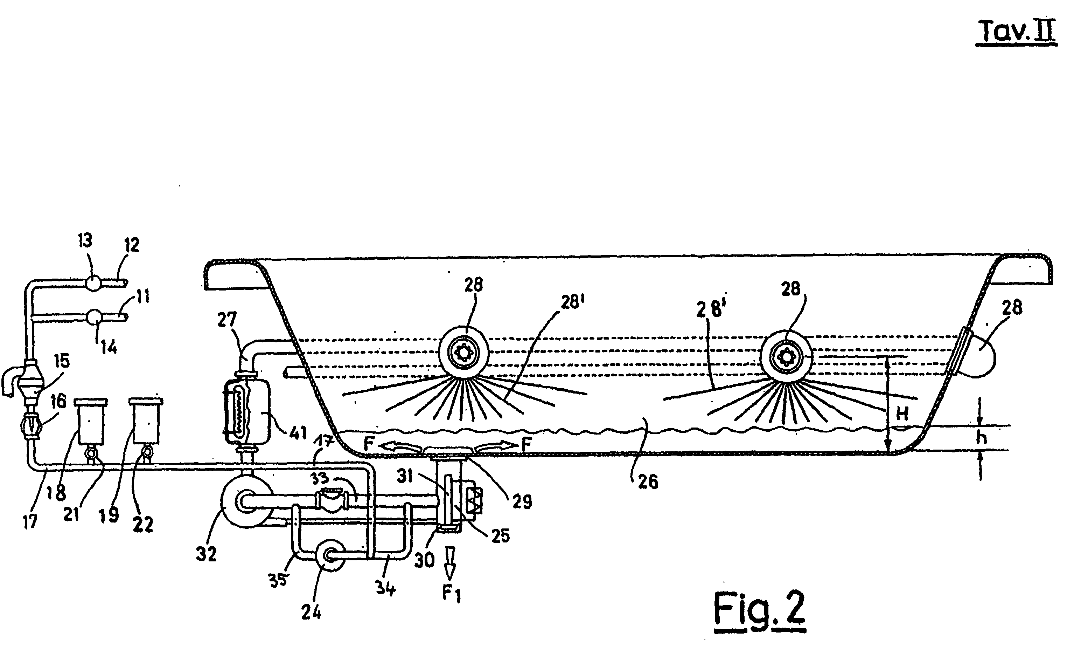

With reference to Figure 2, which shows a modified embodiment compared to that of Figure 1, it will be noted that the delivery pipe 27 incorporates an ultraviolet ray (preferably UVA) device 41 consisting essentially of a lamp of suitable power.

With this device, sterilisation of the water is performed continuously during hydromassage and use is made of the considerable sterilising capacity of the UVA rays.

By adopting this solution it is possible to eliminate the plant disinfecting stage (which will be described in more detail below); consequently, in the plant in Figure 2, the metering reservoirs 18 and 19 have been retained, while the metering reservoir 20 (containing disinfectant) has been eliminated.

However, it is not excluded that the ultraviolet ray device may also be combined with the plant disinfecting operation both in order to achieve greater efficiency or more simply in order to satisfy the psychological need of the user to verify visually the existence of a disinfecting operation.

If we now examine operation of the plant illustrated in Figure 1, it will be noted that the hot or cold or suitably mixed water coming from the inlet pipes 11 and 12 connected to the water mains, flows along the supply pipe 17 as far as the nozzle 25 and, via the inlet nozzle 29, with the drainage nozzle 30 closed, enters the bath 26 as indicated by the arrows F.

The quantity of water inside the bath is predefined and must be limited as far as possible so as to allow subsequently circulation of the fluid for washing and disinfecting the entire water circuit of the system.

The level of the water is therefore fixed at a height h which is situated between the bottom of the bath and the height H of the delivery nozzles 28; advantageously this level may be at a height h from the bottom of the bath, which is less than a 1/3rd of the height H of the delivery nozzles.

The metering reservoir 18 containing a cleaning substance is opened by means of operation of the solenoid valve 21, and a predetermined quantity of cleaning product enters the pipe 17, is mixed with the water and is introduced into the bath 26 via the nozzle 25.

After closing the solenoid valves 13 and 14 of the inlets, the valves 21, 22 and 23 of the metering reservoirs and the valve 31 of the drainage nozzle, and after also setting the delivery nozzles for the washing stage, i.e. generally by assigning them a predetermined aperture or in particular, in the case of the delivery nozzles which are the subject of the aforementioned Patent Applications of the same Applicant, by opening the service channels arranged in a fan-like configuration, the auxiliary pump 24 is activated.

The latter sucks in the additive-containing water from the bath 26, via the suction nozzle 29, and conveys it into the pipe 27 and from the latter to the delivery nozzles 28 which are in an open configuration.

The additive-containing water passes through the nozzle 28 and flows out into the bath 26 in the form of jets which in Figure 1 have been shown in a fan arrangement 28', but which may have other suitable configurations.

The additive-containing water flows down the walls of the bath 26 and reaches the water lying at the bottom of the bath itself so as to be recycled again for a predetermined period of time. Once the washing cycle has been completed, the auxiliary pump 24 stops, the drainage nozzle 30 is opened by means of the solenoid valve 31 and the additive-containing water, which has now been fully used, is discharged from the bath as indicated by the arrow F1.

The same process is repeated with the removal of a quantity of the disinfecting substance from the metering reservoir 19 so as to proceed with disinfecting of the bath and the associated hydraulic system.

At the end of the disinfecting cycle, a rinsing cycle can be suitably performed with water containing no additives, which is taken from the inlets, conveyed into the pipe 17 and introduced into the bath 26 through the nozzle 25.

With the drainage point 30 closed, the water is sucked by the pump 24 and passes from the bath, via the nozzle 29, to the pipe 27 so as to reach the delivery nozzles 28, suitably opened, through which the water flows and passes out into the interior of the bath, accumulating on the bottom so as to be recycled again until completion of the rinsing stage, at the end of which the discharge nozzle is opened and the auxiliary pump is stopped.

The rinsing stage may be followed by a scenting stage, activating a new cycle which is not dissimilar to the washing and disinfecting cycle, with activation of the metering reservoir 20.

While, in the above description, a separate cycle has been envisaged for each washing, disinfecting, scenting and rinsing stage, it is not excluded that a cycle may include several stages, for example washing/disinfecting, or that a cycle may be suppressed, for example the scenting cycle, or the disinfecting cycle when the ultraviolet ray device indicated by 44 in Figure 2 is activated so as to operate continuously during the hydromassage.

It will be noted that the process described and the plant shown fully achieve the aims set.

In fact, in contrast to the known prior art, the delivery nozzles and the interior of the bath are involved in the washing/disinfecting process; moreover, the amount of water used is very small and consequently a smaller quantity of additives is used with positive effects in terms of costs and from an ecological point of view.

It will be noted also that the user is able to verify visually the operations performed during washing, disinfecting and rinsing of the system, before getting into the bath and undergoing the hydromassage.

A further, not insignificant aspect is that all the operations described are performed automatically or sequentially by means of a timer or a computerized unit, making it extremely simple for the user to intervene and activate the process by pressing a single start button.

While the present invention has been described in relation to a preferred embodiment, it is understood that the patent covers all variations and modifications to this embodiment included within the scope of the appended Claims.

## Claims

1. A time-controlled process for cleaning and/or disinfecting a hydromassage system including a bath (26) provided with one or more delivery nozzles (28) arranged at a certain height (H) from the bottom of the bath (26) and a hydraulic circuit having at least one pumping means (24, 32) and a delivery pipe (27) connected to the said nozzles (28), which comprises the steps of:
(a) introducing into said bath (26), through a supply pipe (17) branched off from the water mains and a nozzle (29) arranged at the bottom of the bath, a predetermined quantity of water containing at least one additive which corresponds to a level (h) situated between the bottom of the bath (26) and the said height (H) of the delivery nozzles (28);
(b) activating said pump (24, 32) so as to suck the additive-containing water from the bath (26) through the nozzle (29) arranged at the bottom of the bath (26) and to convey it to each of the said delivery nozzles (28) along said delivery pipe (27);
(c) emitting the additive-containing water from the delivery nozzles (28) so as to let it flow down the walls of the bath (28);
(d) recycling the additive-containing water by the said pump (24, 32) from said nozzle (29) arranged at the bottom of the bath (26) through said hydraulic circuit so as to impinge the internal walls of the bath (26);
(e) discharging the additive-containing water out of the bath (26).

2. Process according to Claim 1, **characterized in that** the said level (h) of the predetermined quantity of additive-containing water is less than 1/3^{rd} of the height (H) of the delivery nozzles (28).

3. Process according to Claim1, **characterized in that** each additive is chosen from among a cleaning substance, a disinfecting substance and a scenting substance.

4. Process according to Claim 1, **characterized in that** each additive is added to the water separately from the other additives.

5. Process according to Claim 1, **characterized in that** various additives are added simultaneously to the water.

6. Process according to Claim 1, **characterized in that** it comprises a step in which the water is sterilized by means of ultraviolet rays during the said recycling step.

7. A hydromassage system which comprises a bath (26), with one or more delivery nozzles (28) arranged at a certain height (H) from the bottom surface of the bath (26); a hydraulic circuit and a timing unit, in which the hydraulic circuit includes :
- a water supply pipe (17) branched off from the water mains (11, 12) and connected via corresponding solenoid valves (21, 22, 23) to at least one metering reservoir (18, 19, 20), each of them containing a different additive,
- a drainage nozzle (30) arranged below the bottom surface of the bath (26) which is operated by a solenoid valve (31),,
- at least one electric pump (24, 32) having its suction side connected to the water supply pipe (17) and its delivery side connected through a delivery pipe (27) to the said delivery nozzles (28), and in which the timing unit is intended for controlling the solenoid valves (21,22,23,31) and electric pump (24,32) of the hydraulic circuit, the *hydromassage system* being **characterized in that** the drainage nozzle (30) is the second and lower part of a nozzle unit (20) comprising as first part an additional nozzle (29) which, under the control of said timing unit, is selectively connected to:
- the water supply line (17) in order to fill the bath (26)
- the suction side of the said pump (24, 32) *in order* to perform the various steps of a cleaning and/or disinfecting process according to any of the Claims 1 to 6,
- the said drainage nozzle (30) in order to ensure the discharge of water out of the bath (26).

8. System according to Claim 7, **characterized in that** a device (41) for generating ultraviolet rays, which are used to sterilize water, is associated to the hydraulic circuit.

9. System according to Claim 8, **characterized in that** the device (41) for generating ultraviolet rays is arranged on the said delivery pipe (27) leading to the delivery nozzles (28).

10. Process according to Claim 1, **characterized in that** the delivery nozzles (28) are adapted to produce fan-like jets of water impinging the internal walls of the bath (26).

## Patentansprüche

1. Ein zeitgesteuerter Prozeß zur Reinigung und/oder Desinfektion eines Hydromassagesystems, das eine Badewanne (26) enthält, die mit einer oder mehrerer Zuführdüsen (28) versehen ist, die in einer bestimmten Höhe (H) von dem Boden der Badewanne (26) aus angeordnet sind, und mit einem hydraulischen Kreislauf, der zumindest eine Pumpe (24, 32) besitzt, sowie ein Förderrohr (27), das mit den Düsen (28) verbunden ist, der die folgenden Schritte aufweist:
(a) Einführen einer vorbestimmten Wassermenge, die zumindest einen Zusatz enthält, die einem Pegel (h) entspricht, der zwischen dem Boden der Badewanne (26) und der Höhe (H) der Zuführdüsen (28) angeordnet ist, durch eine Versorgungsleitung (17), die von den Wasserhauptleitungen und einer Düse (29), die am Boden der Badewanne angeordnet ist, abgezweigt ist;
(b) Aktivieren der Pumpe (24, 32), um das den Zusatz enthaltende Wasser aus der Badewanne (26) durch die Düse (29), die am Boden der Badewanne (26) angeordnet ist, anzusaugen und dieses zu jeder der Zuführdüsen (28) entlang der Zuführleitung (27) zu befördern;
(c) Ausgeben des den Zusatz enthaltenden Wassers aus den Zuführdüsen (28), um es die Wände der Badewanne (28) herunterlaufen zu lassen;
(d) Wiederverwenden des den Zusatz enthaltenden Wassers durch die Pumpe (24, 32) aus der Düse (29), die am Boden der Badewanne (26) angeordnet ist, durch den hydraulischen Kreislauf, um auf die inneren Wände der Badewanne (26) zu treffen;
(e) Auslassen des den Zusatz enthaltenden Wassers aus der Badewanne (26).

2. Prozeß gemäß Anspruch 1, **dadurch gekennzeichnet, daß** der Pegel (h) mit der vorbestimmten Menge an den Zusatz enthaltenden Wassers geringer als ein Drittel der Höhe (H) der Zuführdüsen (28) ist.

3. Prozeß gemäß Anspruch 1, **dadurch gekennzeichnet, daß** jeder Zusatz aus einer Reinigungssubstanz, einer Desinfektionssubstanz und einer Duftsubstanz ausgewählt wird.

4. Prozeß gemäß Anspruch 1, **dadurch gekennzeichnet, daß** jeder Zusatz getrennt von den anderen Zusätzen dem Wasser zugefügt wird.

5. Prozeß gemäß Anspruch 1, **dadurch gekennzeichnet, daß** verschiedene Zusätze gleichzeitig dem Wasser zugefügt werden.

6. Prozeß gemäß Anspruch 1, **dadurch gekennzeichnet, daß** er einen Schritt aufweist, bei dem das Wasser mittels ultravioletter Strahlen während des Wiederverwendungsschrittes sterilisiert wird.

7. Hydromassagesystem, das eine Badewanne (26) aufweist, mit einer oder mehrerer Zuführdüsen (28), die in einer bestimmten Höhe (H) von der Bodenoberfläche der Badewanne (26) angeordnet sind; einen hydraulischen Kreislauf und eine zeitliche Steuerungseinheit, wobei der hydraulische Kreislauf folgendes enthält:
- eine Wasserversorgungsleitung (17), die von den Hauptwasserleitungen (11, 12) abgezweigt ist und über entsprechende Solenoidventile (21, 22, 23) mit wenigstens einem Dosierspeicher (18, 19, 20) verbunden ist, von denen jeder einen unterschiedlichen Zusatz enthält,
- eine Entwässerungsdüse (30), die unter der Bodenoberfläche der Badewanne (26) angeordnet ist, die mit einem Solenoidventil (31) betätigt wird,
- wenigstens eine elektrische Pumpe (24, 32), deren Ansaugseite mit der Wasserversorgungsleitung (17) verbunden ist, und deren Förderseite durch eine Zuführleitung (27) mit den Zuführdüsen (28) verbunden ist,
und wobei die zeitliche Steuerungseinheit zur Steuerung der Solenoidventile (21, 22, 23, 31) und der elektrischen Pumpe (24, 32) des hydraulischen Kreislaufs vorgesehen ist, wobei das Hydromassagesystem **dadurch gekennzeichnet ist, daß** die Entwässerungsdüse (30) der zweite und niedrigere Teil einer Düseneinheit (25) ist, die als einen ersten Teil eine zusätzliche Düse (29) aufweist, die unter der Steuerung der zeitlichen Steuereinheit wahlweise verbunden wird mit:
- der Wasserversorgungsleitung (17) um die Badewanne (26) zu füllen,
- der Ansaugseite der Pumpe (24, 32), um die verschiedenen Schritte eines Reinigungs- und/oder eines Desinfektionsprozesses gemäß einem der Ansprüche 1 bis 6 durchzuführen,
- der Entwässerungsdüse (30), um den Ausstoß des Wassers aus der Badewanne (26) sicherzustellen.

8. System gemäß Anspruch 7, **dadurch gekennzeichnet, daß** eine Vorrichtung (41) zur Erzeugung ultravioletter Strahlen, die verwendet werden, um Wasser zu sterilisieren, zu dem hydraulischen Kreislauf gehören.

9. System gemäß Anspruch 8, **dadurch gekennzeichnet, daß** die Vorrichtung (41) zur Erzeugung ultravioletter Strahlen auf der Zuführleitung (27) angeordnet ist, um zu den Zuführdüsen (28) zu führen.

10. Prozeß gemäß Anspruch 1, **dadurch gekennzeichnet, daß** die Zuführdüsen (28) dazu angepaßt sind, ventilatorähnliche Wasserstrahlen zu erzeugen, die auf die inneren Wände der Badewanne (26) treffen.

## Revendications

1. Processus minuté pour nettoyer et/ou désinfecter un système d'hydromassage comprenant une baignoire (26) équipée d'un ou plusieurs injecteurs (28) agencés à une certaine hauteur (H) à partir du fond de la baignoire (26) et d'un circuit hydraulique possédant au moins une pompe (24, 32) et une tuyauterie d'injection (27) raccordée auxdits injecteurs (28), lequel processus comprend les étapes suivantes :
(a) introduire dans ladite baignoire (26) via une tuyauterie d'alimentation (17) débranchée des conduites principales d'eau et via une bouche (29) se trouvant au fond de la baignoire, une quantité d'eau prédéterminée contenant au moins un additif qui correspond à un niveau (h) situé entre le fond de la baignoire (26) et ladite hauteur (H) des injecteurs (28) ;
(b) activer ladite pompe (24, 32) de telle sorte qu'elle aspire l'eau contenant l'additif de la baignoire (26) par la bouche (29) se trouvant au fond de la baignoire (26) et qu'elle l'achemine vers chaque injecteur (28) le long de ladite tuyauterie d'injection (27) ;
(c) sortir l'eau contenant l'additif par les injecteurs (28) de telle sorte qu'elle puisse s'écouler le long des parois de la baignoire (28) ;
(d) recycler l'eau contenant l'additif par ladite pompe (24, 32) à partir de ladite bouche (29) se trouvant au fond de la baignoire (26) à travers ledit circuit hydraulique de telle sorte qu'elle retombe sur les parois internes de la baignoire (26) ;
(e) évacuer l'eau contenant l'additif hors de la baignoire (26).

2. Processus selon la revendication 1, **caractérisé en ce que** ledit niveau (h) de la quantité prédéterminée d'eau contenant l'additif est inférieur au tiers de la hauteur (H) des injecteurs (28).

3. Processus selon la revendication 1, **caractérisé en ce que** chaque additif est choisi parmi un produit de nettoyage, un produit désinfectant et un produit désodorisant.

4. Processus selon la revendication 1, **caractérisé en ce que** chaque additif est ajouté à l'eau séparément des autres additifs.

5. Processus selon la revendication 1, **caractérisé en ce que** plusieurs additifs sont ajoutés à l'eau simultanément.

6. Processus selon la revendication 1, **caractérisé en ce qu'**il comprend une étape au cours de laquelle l'eau est stérilisée au moyen de rayons ultraviolets pendant ladite étape de recyclage.

7. Système d'hydromassage comprenant une baignoire (26) équipée d'un ou de plusieurs(s) injecteurs (28) situés à une certaine hauteur (H) du fond de la baignoire (26), un circuit hydraulique et une minuterie, dans lequel le circuit hydraulique comprend :
- une tuyauterie d'alimentation en eau (17) débranchée des conduites principales d'eau (11, 12) et raccordée par le biais des électrovannes correspondantes (21, 22, 23) à au moins un réservoir de dosage (18, 19, 20), chacun d'entre eux contenant un additif différent,
- une bouche d'évacuation (30) située en dessous du fond de la baignoire (26) qui est actionnée par une électrovanne (31),
- au moins une pompe électrique (24, 32) dont le côté d'aspiration est raccordé à la tuyauterie d'alimentation en eau (17) et le côté d'injection est raccordé par le biais d'une tuyauterie d'injection auxdits injecteurs (28),
et dans lequel la minuterie a pour fonction de contrôler les électrovannes (21, 22, 23, 31) et la pompe électrique (24, 32) du circuit hydraulique, le système d'hydromassage étant **caractérisé en ce que** la bouche d'évacuation (30) est la seconde partie et la partie inférieure d'un ensemble formant bouche (25) comprenant comme première partie une bouche annexe (29) qui, sous le contrôle de ladite minuterie, est sélectivement raccordée à :
- la tuyauterie d'alimentation en eau (17) afin de remplir la baignoire (26),
- le côté d'aspiration de ladite pompe (24, 32) afin de réaliser les différentes étapes d'un processus de nettoyage et/ou de désinfection selon l'une quelconque des revendications 1 à 6,
- ladite bouche d'évacuation (30) afin d'assurer l'évacuation de l'eau hors de la baignoire (26).

8. Système selon la revendication 7, **caractérisé en ce qu'**un dispositif (41) destiné à générer des rayons ultraviolets, utilisés pour stériliser l'eau, est associé au circuit hydraulique.

9. Système selon la revendication 8, **caractérisé en ce que** le dispositif (41) destiné à générer des rayons ultraviolets est situé sur ladite tuyauterie d'injection (27) conduisant aux injecteurs (28).

10. Processus selon la revendication 1, **caractérisé en ce que** les injecteurs (28) sont adaptés pour produire des jets d'eau en éventail retombant sur les parois internes de la baignoire (26).
